(19) Europäisches Patentamt
European Patent Office
Office européen des brevets

(11) Veröffentlichungsnummer: **0 437 846 A2**

(12) **EUROPÄISCHE PATENTANMELDUNG**

(21) Anmeldenummer: **90125643.8**

(51) Int. Cl.$^5$: **C07K 9/00**, A61K 37/02

(22) Anmeldetag: **28.12.90**

(30) Priorität: **06.01.90 DE 4000236**
**27.02.90 DE 4006141**

(43) Veröffentlichungstag der Anmeldung:
**24.07.91 Patentblatt 91/30**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Vértesy, Lászl , Dr.**
**Eppenhainer Weg 6**
**W-6239 Eppstein-Taunus(DE)**
Erfinder: **Seibert, Gerhard, Prof.**
**Glaeserweg 21**
**W-6100 Darmstadt(DE)**

(54) **Decaplaninsalzlösungen, deren Herstellung und deren Verwendung, sowie kristalline Decaplaninsalze, deren Herstellung und deren Verwendung.**

(57) Decaplaninsalzlösungen, deren Herstellung und deren Verwendung sowie kristalline Decaplaninsalze, deren Herstellung und deren Verwendung

Wäßrige Decaplaninsalzlösungen eignen sich zur Verabreichung des Decaplanins in Form von Injektionslösungen sowie zur Reinigung von Decaplanin. Kristallines Decaplanin und seine Salze besitzen Bedeutung für die Reinigung des Decaplanins und seiner Salze und eignen sich als Vorstufe einer Darreichungsform.

EP 0 437 846 A2

Xerox Copy Centre

# DECAPLANINSALZLÖSUNGEN, DEREN HERSTELLUNG UND DEREN VERWENDUNG, SOWIE KRISTALLINE DECAPLANINSALZE, DEREN HERSTELLUNG UND DEREN VERWENDUNG

Die vorliegende Erfindung betrifft Decaplaninsalzlösungen, deren Herstellung und deren Verwendung sowie kristalline Decaplaninsalze, deren Herstellung und deren Verwendung.

Decaplanin, eine Verbindung der Formel I

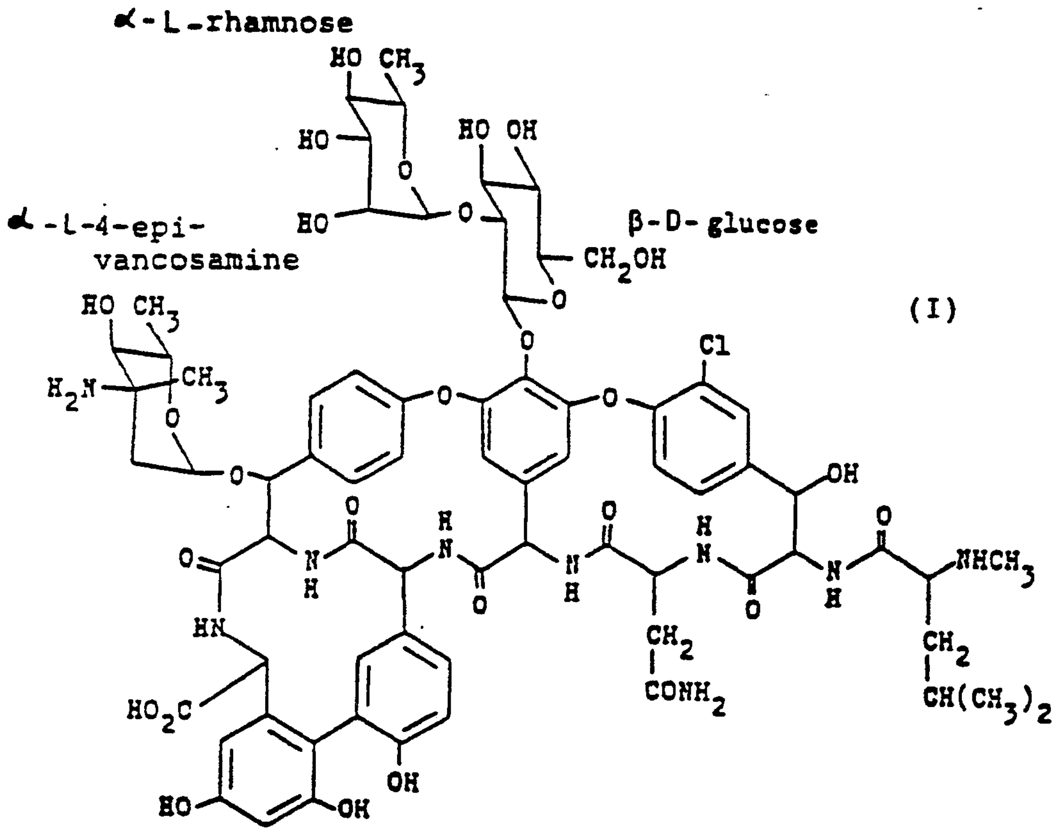


(I)

ist Gegenstand der Europäischen Patentanmeldung 89 115 519.4; einige amorphe Decaplaninsalze wie das HCl-, $H_2SO_4$-, $CF_3COOH$- und $CH_3COOH$-Salz sind in der genannten Patentanmeldung ebenfalls erwähnt. Es besteht ein Bedarf an wäßrigen Decaplaninwirkstofflösungen, da Decaplanin ein Antibiotikum des Vancomycintyps ist, somit oral nicht resorbierbar ist und deshalb zweckmäßigerweise per Injektion verabreicht wird. Decaplanin selbst ist relativ wenig wasserlöslich. Das Zitrat, das Glukonat, das Sulfat und das Hydrochlorid sind ebenfalls nicht ausreichend löslich (Löslichkeit zum Teil deutlich unter 5 %). Darüber hinaus zeigen viele Decaplaninsalze in vergleichsweise dünnen Lösungen eine Neigung zum Gelieren. Decaplaninsalze solcher Säuren lassen sich zwar in Wasser lösen, die Lösung bleibt aber nicht klar und dünnflüssig, sondern sie wird zähflüssig. Dieses Gelieren läßt sich nicht einmal durch Erhöhung der Temperatur beseitigen; im Gegenteil, der Effekt ist bei Kühlschranktemperatur sogar geringer als bei Raumtemperatur oder 37 °C. Derartige Lösungen, die für die Injektionstechnik völlig ungeeignet sind, bilden sich z.B. mit den folgenden Säuren: Milchsäure, Maleinsäure, Malonsäure, Gluconsäure, Bernsteinsäure und Salzsäure.

Überraschenderweise wurde nun gefunden, daß die Salze des Decaplanins mit der Essigsäure, mit der Phosphorsäure und mit der Weinsäure sich durch ein gutes Löslichkeitsverhalten auszeichnen. Lösungen dieser Salze sind hervorragend zur Injektion des Decaplaninwirkstoffs geeignet und zeichnen sich wie das Decaplanin selbst durch eine sehr geringe Toxizität und gute Verträglichkeit aus. Dies ist besonders überraschend, weil ein anderes Glycopeptidantibiotikum, das Vancomycin, gemeinhin als Hydrochlorid d.h. in einer relativ wenig löslichen Form angeboten wird, die zudem schlecht verträglich ist, weswegen sie während einer langen Zeit von z.B. einer halben Stunde injiziert werden muß. Die Nebenwirkungen dieses Vancomycinsalzes sind lokale Nekrosen, das "red man-Syndrom" sowie Oto- und Nephrotoxizität. Derartige Nebenwirkungen können bei den zuletztgenannten Decaplaninsalzen nicht beobachtet werden.

Erfindungsgegenstand ist demzufolge eine wäßrige Lösung eines Decaplaninsalzes, ausgewählt aus der

Gruppe der Decaplaninacetate, Decaplaninphosphate und Decaplanintartrate. Aus dieser Gruppe sind die Decaplaninacetate besonders bevorzugt. Die erfindungsgemäßen wässrigen Lösungen haben wie bereits erläutert den Vorteil, daß in ihnen das jeweilige Salz in relativ hoher Konzentration vorliegen kann, ohne daß das betreffende Salz ausfällt. So ist z.B. das Decaplaninacetat in bis zu ca. 20 Gew.-%iger Konzentration löslich; die Lösung bleibt auch bei dieser Konzentration überraschenderweise dünnflüssig, physiologisch gut verträglich und stabil. So zeigt sich z.B. in einer 0,2 %igen wäßrigen Decaplaninmonoacetatlösung bei einer 20 stündigen Temperaturbelastung von 80 °C nur ein Wirkstoffverlust von weniger als 5 %; Vancomycinhydrochlorid hingegen weist bei einer analogen Behandlung nur noch einen Gehalt von etwa 25 % Vancomycinhydrochlorid auf. Die Instabilität des Vancomycins resultiert aus seiner Umwandlung zum sogenannten CDP (vgl. F. Sztaricskai und R. Bognar, Chemistry of the Vancomycin Group of Antibiotics. In:Recent Developments in the Chemistry of Natural Carbon Compounds, Vol. 10, PP 93-201, 1984, Akademiai Kiado, Budapest, 1983). Es ist besonders überraschend, daß die erfindungsgemäßen Decaplaninsalze diese Umwandlung nicht in diesem Ausmaß erleiden. Weiterhin besonders überraschend ist die bereits erwähnte, im Gegensatz zum Vancomycinhydrochlorid stehende gute lokale Verträglichkeit der erfindungsgemäßen wäßrigen Decaplaninsalzlösungen, die ohne besondere Vorsichtsmaßnahmen - wie langsame Injektion - injiziert werden können und auch subcutan verabreichbar sind.

Die erfindungsgemäßen Decaplaninsalzlösungen können einen unterschiedlichen Gehalt an Decaplaninsalz aufweisen. Bevorzugt ist ein Gehalt von bis zu 20 Gew.-%, besonders bevorzugt ein Gehalt von 15 Gew.-% insbesondere ein Gehalt von bis zu 10 Gew.-% des Decaplaninsalzes.

Das jeweils zum Einsatz kommenden Decaplaninsalz muß nicht unbedingt Ergebnis einer stöchiometrischen Reaktion sein.

Das Decaplanin kann mit einem Unterschuß oder Überschuß an Säure umgesetzt werden. Im Falle des Decaplaninacetats ist das Umsetzungsprodukt aus 1 Mol Decaplanin mit 0,5 - 3 Mol Essigsäure bevorzugt, besonders bevorzugt sind 0,8 - 2 Mol Essigsäure auf 1 Mol Decaplanin. Die Stöchiometrie des Produktes läßt sich bei der Kristallisation einstellen, indem der Anfangs-pH des Kristallisationsansatzes auf einen bestimmten Wert eingestellt wird. Die folgende Tabelle zeigt beispielshaft den Zusammenhang zwischen dem pH-Wert der Kristallisation und dem Acetatanteil im gewonnen, getrockneten Kristallisat im Falle des Decaplaninacetates.

| pH-Wert der Kristallisation | 4,0 | 4,3 | 4,6 | 4,9 | 5,1 | 5,3 | 5,5 | 5,8 | 6,1 |
|---|---|---|---|---|---|---|---|---|---|
| rel. Acetatanteil in Mol-Prozent im Endprodukt | 181% | 150% | 110% | 97% | 90% | 86% | 80% | 79% | 75% |

Die Decaplaninacetatfestsubstanz kann mit beliebigem Acetatanteil auch durch Gefriertrocknen erhalten werden. Derartige Lyophilisate haben den Vorteil, daß sie sich besonders schnell lösen. Die folgende Tabelle zeigt beispielhaft den Zusammenhang zwischen dem Anfangs-pH-Wert der Gefriertrocknung und den relativen Mol-Prozenten des Acetates im Lyophilisat ebenfalls im Falle des Decaplaninacetates.

| pH-Wert der Gefriertrocknung | 4,0 | 4,3 | 4,6 | 4,9 | 5,2 | 5,5 | 5,8 |
|---|---|---|---|---|---|---|---|
| rel. Acetatanteil in Mol-Prozent im Lyophilisat | 175 | 150 | 110 | 100 | 90 | 85 | 80 |

Der Acetatanteil des Decaplaninacetatsalzes ist für die Löslichkeit des Produktes wichtig. Mit zunehmendem Acetatanteil steigt im allgemeinen die Löslichkeit des Präparates.

Gegenstand der vorliegenden Erfindung sind weiterhin Injektionslösungen die einen Gehalt an Decaplaninsalzen aufweisen. Die Decaplaninsalzkonzentration in den Injektionslösungen ist vorzugsweise in den

Bereichen zu wählen, die für die wäßrigen Decaplaninsalzlösungen bereits genannt wurden. Die erfindungsgemäßen Injektionslösungen können weiterhin übliche Hilfs- oder Zusatzstoffe enthalten. Als Hilfs- oder Zusatzstoffe seien bspw. erwähnt Polyglykole, Ethanol, Puffersubstanzen wie z.B. N-, N-Dibenzylethylendiamin, Diethanolamin, Ethylendiamin, N- Benzylphenethylamin, Diethylamin, Tris-(hydroxymethyl)-aminomethan. Weitere Zusatzstoffe können sein z.B. NaCl, Phosphatpuffer, Essigsäure, Natriumbicarbonat oder Natriumcarbonat.

Die bevorzugte Tagesdosis der Decaplaninsalze für einen erwachsenen Menschen, die mit den erfindungsgemäßen Injektionslösungen zu verabreichen ist, liegt im Bereich von 250 mg bis ca. 1500 mg. Die besonders bevorzugte Tagesdosis liegt im Bereich von ca. 1000 mg für einen erwachsenen Menschen. Die jeweilige Tagesdosis an Decaplaninsalz wird vorzugsweise in 2-3 Einzeldosen verabreicht. Bei Bedarf kann von den vorgeschlagenen Dosierungen abgewichen werden, insbesondere kann die Tagesdosis kurzfristig erheblich erhöht werden.

Zum Erfindungsgegenstand gehört weiterhin ein Verfahren zur Herstellung der Decaplaninsalzlösungen, das dadurch gekennzeichnet ist, daß die Herstellung unter Verwendung von Adsorptionsharzen, Ionenaustauschern oder unter Anwendung der Dialyse und der Ultrafiltration erfolgt. Bei der Verwendung von Adsorptionsharzen, insbesondere für das Decaplaninacetat ist es vorteilhaft, Adsorptionsharze wie z.B. Diaion® HP-20 (hochgradig poröses Adsorptionsharz basierend auf einem Polystyrol-Divinylbenzol Kopolymer, Mitsubishi Chemical Inc.) oder Amberlite® XAD (poröses Adsorptionsharz basierend auf Polystyrol oder Acrylsäureester, Rohm & Haas, Philadelphia, USA) zu benutzen. Man stellt hierbei vorzugsweise den pH-Wert einer beliebigen Decaplaninsalzlösung auf 8,1, den pH des isoelektrischen Punktes ein, und trägt auf eine salzfreie Adsorptionsharzsäule auf. Anschließend wird die Säule zur Entfernung der Fremdionen mit ionenfreiem Wasser gewaschen. Die Gewinnung des nun entsalzten Antibiotikums erfolgt durch Elution mit geeigneten organischen Lösungsmitteln oder Wasser/Lösungsmittelgemischen. Geeignete Lösungsmittel sind z.B. niedere Alkohole, Aceton, Acetonitril etc.. Die salzfreie Verbindung kann dann mit entsprechenden Mengen Säure, z. B. Essigsäure, Phosphorsäure oder Weinsäure versetzt werden. Nach Gefriertrocknung oder gegebenenfalls Kristallisation erhält man das feste Salz Das Abziehen des gegebenenfalls vorhandenen organischen Lösungsmittels ergibt direkt die gewünschte wäßrige Salzlösung.

Bei Anwendung der Ionenaustauschertechnik zur Herstellung der genannten Decaplaninsalze werden vorzugsweise bekannte Kationenaustauscher benutzt. Der Kationenaustauscher sowie die Decaplaninlösung werden auf einen pH-Wert vorzugsweise zwischen 3 und 7 eingestellt und die Decaplaninlösung wird auf den Kationenaustauscher gegeben. Nachdem mit Wasser gewaschen wurde, wird z. B. mit Acetat-, Phosphat- oder Tartratlösung hoher Konzentration eluiert und das entsprechende Eluat vorzugsweise durch Dialyse oder Ultrafiltration auf die gewünschte Konzentration gebracht.

Es ist weiterhin möglich nur mit Hilfe der Dialyse und der Ultrafiltration z. B. das Acetat, das Phosphat oder das Tartrat des Decaplanins zu erhalten. Man dialysiert gegen Acetat, Phosphat bzw. Tartrat oder verdünnt das Retenat der Ultrafiltration mit entsprechenden Salzen des gewünschten Anions bis im Filtrat kein Fremdion mehr nachweisbar ist. Die so erhaltenen Lösungen können anschließend zur Kristallisation gebracht werden.

Zum Erfindungsgegenstand gehört weiterhin die Verwendung von Decaplaninsalzlösungen zur Reinigung des Decaplanins sowie ein Verfahren zur Reinigung von Decaplanin, das dadurch gekennzeichnet ist, daß man das Decaplanin in eines seiner Salze in wässriger Lösung überführt, das Decaplaninsalz durch Temperaturänderung oder durch Zugabe eines geeigneten Lösungsmittels zum Kristallisieren bringt und anschließend gegebenenfalls das Decaplaninsalz in Decaplanin oder in ein anderes Decaplaninsalz umwandelt. Geeignete Lösungsmittel sind z.B. n- oder iso-Propanol, Ethanol, Methanol oder Aceton. Das Lösungsmittel wird vorzugsweise bis zu einem Gehalt von 5 bis 90 % zugegeben, besonders bevorzugt zu einem Gehalt von 10 bis 50 %. Im Falle des Decaplaninmonoacetates ist es besonders bevorzugt von einer ca. 8 %igen wässrigen Decaplaninacetatlösung auszugehen und bei Raumtemperatur 10 Vol.-% Ethanol hinzuzugeben. Nach dem Auftreten von Kristallen wird der Ethanolanteil zweckmäßigerweise allmählich auf 25 % erhöht und der Ansatz bei einer niedrigen Temperatur, vorzugsweise ca. 4 °C, mehrere Tage stehen gelassen. Die auftretende Kristallmasse kann z.B. durch Abfiltrieren oder Abzentrifugieren abgetrennt werden.

Weiterhin funktioniert das beschriebene Verfahren auch mit reinem Decaplanin, wenn man in der Nähe des isoelektrischen Punktes (pH = 8,1) arbeitet und das kristalline Decaplanin als inneres Salz erhält.

Die Einführung einer Kristallisationsstufe ist für die Reinigung eines Antibiotikums von der Größe des Decaplanins von besonderer Bedeutung. Bei der fermentativen Herstellung des Decaplanins können Begleitstoffe wie z. B. andere Peptide oder Enzyme entstehen, die nur schwer vom Decaplanin abgetrennt werden können. Derartige nicht abgetrennte Begleitstoffe können aber die Qualität des Antibiotikums drastisch verschlechtern, weil z. B. begleitende Peptide nach der Verabreichung des Antibiotikums zu

allergischen Reaktionen führen oder z. B. Enzyme das Antibiotikum verändern können. Mittels der Kristallisation läßt sich das Decaplanin zu Arzneimittelqualität reinigen.

Das beschriebene Reinigungsverfahren führt zu kristallinen Decaplaninsalzen und kristallinem Decaplanin, die in dem vorliegenden Beschreibungstext erstmals beschrieben werden und ebenfalls zum Gegenstand der vorliegenden Erfindung gehören. Die Gewinnbarkeit von kristallinen Decaplaninsalzen und kristallinem Decaplanin ist an sich überraschend, da Glycopeptide, zu denen auch das Decaplanin gehört, und deren Salze im allgemeinen nur schwer kristallisierbar sind. Die beschriebenen kristallinen Decaplaninsalze und das kristalline Decaplanin fallen in besonders reiner Form an und sind insbesondere bei der Herstellung von besonders reinem Decaplanin und dessen Salzen von Bedeutung. Von besonderem Interesse sind in diesem Zusammenhang die kristallinen Citrate, Acetate, Phosphate, Glucuronate, Tartrate, Sulfate, Succinate sowie das kristalline reine Decaplanin, die sich wie bereits beschrieben herstellen lassen. Von ganz besondererBedeutung sind das kristalline Citrat und das kristallineAcetat des Decaplanins.

Die genannten kristallinen Decaplaninsalze haben aber nicht nur Bedeutung bei der Reinigung des Decaplanins und seinen Salzen, sie haben auch Vorteile, insbesondere hinsichtlich ihrer Stabilität und eignen sich somit als Handelsform und als Vorstufe von Darreichungsformen; z.B. können sie in kristalliner Form bereitgestellt werden, um vor der eigentlichen Applikation aufgelöst zu werden. Als besonders vorteilhaft sind in diesem Zusammenhang kristalline Decaplaninacetate anzusehen.

Durch die nachfolgenden Ausführungsbeispiele soll die Erfindung näher erläutert werden.

**Beispiel 1**

Gewinnung der Acetatform des Decaplanins mittels Kationenaustauscher

Eine 10 ml-Chromatographie-Säule wird mit CM-Sepharose® FF (Pharmacia, Schweden) gefüllt und mit Na-acetat-puffer auf pH 4,0 eingestellt. Dann werden 100 mg halbreines Decaplanin, erhalten z. B. entsprechend Beispiel 2-4 der EP 89 115 519.4 (Fermentation des Mikroorganismus HIL Y-8636910 (DSM 4763), Isolation und Anreicherung des Decaplanins mittels chromatographischer Methoden), in 10 ml Wasser gelöst, auf den Träger aufgetragen und mit 30 ml destilliertem Wasser nachgewaschen. Anschließend legt man einen 10 bis 500 mM Natriumacetat-gradienten, pH 4,0, an. Mit 210-230 mM-Na-acetat wird das Decaplanin als Acetat von dem Ionenaustauscher eluiert. Die entsprechenden Fraktionen werden vereinigt und gegen bidest. Wasser dialysiert. Ihre Gefriertrocknung ergibt 102 mg Decaplaninacetat mit 1,6 Mol Acetat pro Mol Decaplanin.

**Beispiel 2**

Gewinnung der Acetatform des Decaplanins am Adsorptionsharz Diaion® HP-20

Auf 25,5 1 regeneriertes Diaion® HP-20 werden 1,1 kg halbreines Decaplanin, gelöst in 25 l Wasser, bei einem pH-Wert von 8,1 aufgetragen. Danach wäscht man die Säule mit entsalztem Wasser. Hierbei steigt zunächst die Leitfähigkeit im Säulenausfluß auf über 20 mS/cm, um dann in den Fraktionen nach dem Durchlauf auf unter 2 mS/cm zu sinken. Das Decaplanin in der isoelektrischen Form wird nun mit einem 0 bis 50 % Isopropanol enthaltenden Gradienten eluiert. Die Decaplanin-enthaltenden Fraktionen haben eine niedrige Leitfähigkeit von unter 0;1 mS/cm. Sie werden gesammelt und mit Eisessig auf pH 4,8 gestellt. Man erhält 61 l Lösung mit 1 kg Decaplaninacetat.

**Beispiel 3**

Gefriertrocknung des Decaplaninacetats

1 l der nach Beispiel 2 gewonnenen Decaplaninacetatlösung wird 2 Tage gefriergetrocknet. Es resultieren 15,5 g Decaplaninmonoacetat in 98 %iger Reinheit.

**Beispiel 4**

Kristallisation des Decaplaninacetates

60 l der nach Beispiel 2 gewonnenen Decaplaninacetatlösung werden im Vakuum auf 35 l eingeengt. Anschließend konzentriert man durch Ultrafiltration weiter auf 12 l. Das so erhaltene Konzentrat wird nun bei

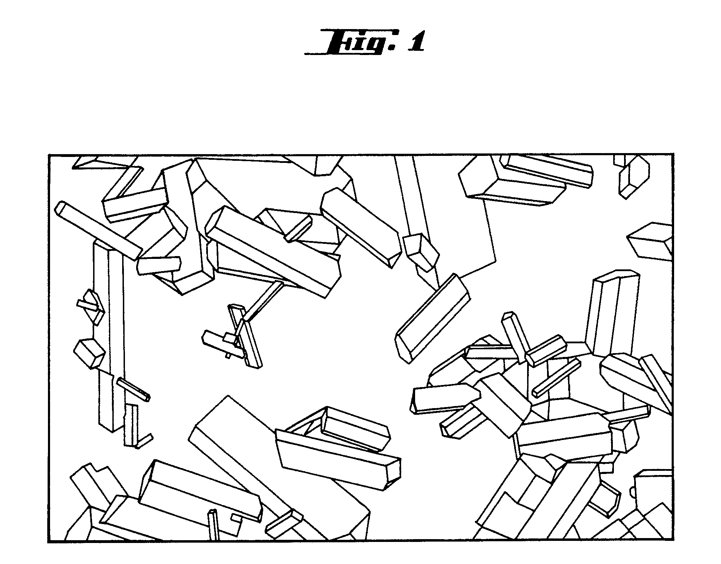

Raumtemperatur langsam mit 12 l Isopropanol versetzt. Nach dem Auftreten von Kristallen wird auf + 1 °C gekühlt und 1 Tag stehen gelassen. Nach dieser Zeit erhöht man den Isopropanolanteil allmählich auf 3 l und läßt weitere 2 Tage stehen. Anschließend wird die Kristallmasse abfiltriert und im Vakuum getrocknet. Man erhält 810 g Decaplaninmonoacetat in 99,4 %iger Reinheit. Die Debye-Scherrer-Aufnahmen, des kristallinen Decaplaninacetats ($CuK_\alpha$) zeigen Reflexe, die sich mit der orthorhombischen Zelle a = 19,34, b = 33,52 und c = 36,18 Angström indizieren lassen. Die drei stärksten Reflexe liegen bei 4,90 (0 0 2), 5,30 (1 1 0) und 7,00° 2 Theta (1 2 0). Die Figur 1 zeigt eine Fotografie von Decaplaninmonoacetat-Kristallen.

**Beispiel 5**

Herstellung einer Decaplaninacetatlösung

10,2 g des nach Beispiel 3 gewonnenen Gefriertrocknungsproduktes werden in 90 ml Wasser gelöst. Sie ergeben 100 ml einer Flüssigkeit mit einer Viskosität von weniger als 3 Centipoise (cP) bei 20 °C.

**Beispiel 6**

Herstellung einer Decaplanindiacetatlösung

10 g des nach Beispiel 3 gewonnenen Produktes werden in 40 ml Wasser aufgenommen und durch Zugabe von 380 g Eisessig eine Decaplanindiacetatlösung hergestellt. Die Lösung hat einen pH-Wert von 4,2. Ihre Viskosität beträgt weniger als 3 cP bei 20 °C.

**Beispiel 7**

Kristallisation des Decaplaninmonocitrates

Man verfährt wie im Beispiel 2 beschrieben, stellt jedoch das HP-20-Eluat mit Citronensäure auf pH 4,7, konzentriert die Lösung durch Ultrafiltration auf 12 l und läßt die Lösung kalt stehen. Nach einigen Stunden entsteht eine Kristallmasse, die nach Erhöhen des Isopropanolanteiles auf 35 % vervollständigt wird. Sie wird gesammelt und getrocknet. Ausbeute: 830 g in 99,6 % Reinheit.

**Patentansprüche**

1. Eine wäßrige Lösung eines Decaplaninsalzes, ausgewählt aus der Gruppe Decaplaninacetat, Decaplaninphosphat und Decaplanintartrat.

2. Eine Lösung gemäß Anspruch 1, dadurch gekennzeichnet, daß das Decaplaninsalz ein Decaplaninacetat ist.

3. Eine Lösung gemäß den Ansprüchen 1 oder 2, dadurch gekennzeichnet, daß die Lösung bis zu 20 Gew.-%, vorzugsweise bis zu 15 Gew.-%, insbesondere bis zu 10 Gew.-% eines Decaplaninsalzes enthält.

4. Eine Lösung gemäß einem oder mehreren der Ansprüche 1-3, dadurch gekennzeichnet, daß das Decaplaninsalz hergestellt wurde durch Umsetzung von 0,5-3 mol, vorzugsweise 0,8-2 mol Säure mit 1 mol Decaplanin.

5. Eine Injektionslösung enthaltend eine wäßrige Decaplaninsalzlösung gemäß einem oder mehreren der Ansprüche 1-4.

6. Verwendung einer Decaplaninsalzlösung gemäß einem oder mehreren der Ansprüche 1-4 zur Herstellung einer Injektionslösung gemäß Anspruch 5.

7. Verfahren zur Herstellung einer Injektionslösung gemäß Anspruch 5, dadurch gekennzeichnet, daß eine wäßrige Decaplaninsalzlösung gegebenenfalls mit üblichen Hilfs- oder Zusatzstoffen in eine geeignete Dosierungsform gebracht wird.

8. Verfahren zur Herstellung von Decaplaninsalzlösungen, dadurch gekennzeichnet, daß die Herstellung unter Verwendung von Adsorptionsharzen, Ionenaustauschern oder unter Anwendung der Dialyse und der Ultrafiltration erfolgt.

9. Verwendung von Decaplaninsalzlösungen zur Reinigung von Decaplanin.

10. Verfahren zur Reinigung von Decaplanin bzw. seiner Salze, dadurch gekennzeichnet, daß man das Decaplanin in eines seiner Salze in wäßriger Lösung überführt, das Decaplaninsalz durch Zugabe eines geeigneten Lösungsmittels zum Ausfallen bringt und anschließend gegebenenfalls das Decaplaninsalz in Decaplanin umwandelt.

11. Kristalline Salze des Decaplanins.

12. Kristalline Salze des Decaplanins gemäß Anspruch 11, dadurch gekennzeichnet, daß sie kristalline Citrate, Acetate, Phosphate, Glucuronate, Tartrate, Sulfate, Succinate oder das innere Salz des Decaplanins sind.

13. Kristalline Salze des Decaplanins gemäß Anspruch 11 oder 12, dadurch gekennzeichnet, daß sie Acetate oder Citrate sind.

14. Verwendung kristalliner Decaplaninsalze nach den Ansprüchen 11 - 13 als Vorstufe einer Darreichungsform.

15. Verfahren zur Herstellung kristalliner Salze des Decaplanins gemäß den Ansprüchen 11-13, dadurch gekennzeichnet, daß man das Decaplanin in eines seiner Salze in wäßriger Lösung überführt und durch Temperaturänderung oder durch Zugabe eines geeigneten Lösungsmittel unter geeigneten Bedingungen zur Kristallisation bringt und die erhaltenen Kristalle nach bekannten Methoden isoliert .

**Patentansprüche für folgende Vertragsstaaten: ES und GR**

1. Ein Verfahren zur Herstellung einer wäßrigen Lösung eines Decaplaninsalzes, ausgewählt aus der Gruppe Decaplaninacetat, Decaplaninphosphat und Decaplanintartrat, dadurch gekennzeichnet, daß das Decaplaninsalz in Wasser gelöst wird.

2. Verfahren gemäß Anspruch 1, dadurch gekennzeichnet, daß das Decaplaninsalz ein Decaplaninacetat ist.

3. Verfahren gemäß den Ansprüchen 1 oder 2, dadurch gekennzeichnet, daß die Lösung bis zu 20 Gew.-%, vorzugsweise bis zu 15 Gew.-%, insbesondere bis zu 10 Gew.-% eines Decaplaninsalzes enthält.

4. Verfahren gemäß einem oder mehreren der Ansprüche 1-3, dadurch gekennzeichnet, daß das Decaplaninsalz hergestellt wurde durch Umsetzung von 0,5-3 mol, vorzugsweise 0,8-2 mol Säure mit 1 mol Decaplanin.

5. Verwendung einer Decaplaninsalzlösung gemäß einem oder mehreren der Ansprüche 1-4 zur Herstellung einer Injektionslösung.

6. Verfahren zur Herstellung einer Injektionslösung gemäß Anspruch 5, dadurch gekennzeichnet, daß eine wäßrige Decaplaninsalzlösung gegebenenfalls mit üblichen Hilfs- oder Zusatzstoffen in eine geeignete Dosierungsform gebracht wird.

7. Verfahren zur Herstellung von Decaplaninsalzlösungen, dadurch gekennzeichnet, daß die Herstellung unter Verwendung von Adsorptionsharzen, Ionenaustauschern oder unter Anwendung der Dialyse und der Ultrafiltration erfolgt.

8. Verwendung von Decaplaninsalzlösungen zur Reinigung von Decaplanin.

9. Verfahren zur Reinigung von Decaplanin bzw. seiner Salze, dadurch gekennzeichnet, daß man das

Decaplanin in eines seiner Salze in wäßriger Lösung überführt, das Decaplaninsalz durch Zugabe eines geeigneten Lösungsmittels zum Ausfallen bringt und anschließend gegebenenfalls das Decaplaninsalz in Decaplanin umwandelt.

**10.** Verfahren gemäß Anspruch 9, dadurch gekennzeichnet, daß das Decaplaninsalz kristallines Citrat, Acetat, Phosphat, Glucuronat, Tartrat, Sulfat, Succinat oder das innere Salz des Decaplanins ist.

**11.** Verwendung kristalliner Decaplaninsalze nach dem Anspruch 10 als Vorstufe einer Darreichungsform.

**12.** Verfahren zur Herstellung kristalliner Salze des Decaplanins gemäß Anspruch 10, dadurch gekennzeichnet, daß man das Decaplanin in eines seiner Salze in wäßriger Lösung überführt und durch Temperaturänderung oder durch Zugabe eines geeigneten Lösungsmittels unter geeigneten Bedingungen zur Kristallisation bringt und die erhaltenen Kristalle nach bekannten Methoden isoliert

Fig. 1